# EUROPÄISCHE PATENTANMELDUNG

(11) **EP 1 101 466 A2**
(43) Veröffentlichungstag der Anmeldung: **23.05.2001**
(21) Anmeldenummer: 00123085.3
(22) Anmeldetag: 25.10.2000
(51) Int. Cl.: A61F 11/00

(54) **Röhrchen zur Belüftung des Mittelohrs**

(30) Priorität: 16.11.1999 DE 29920064 U
(71) Anmelder: Heinz Kurz GmbH Medizintechnik, 72144 Dusslingen (DE)
(72) Erfinder: Kurz, Heinz, 72144 Dusslingen (DE)
(74) Vertreter: Möbus, Daniela, Dr.-Ing.

(57) **Zusammenfassung**

Ein Röhrchen zur Belüftung des Mittelohrs, das einen vorderen kegelförmigen Flansch (12) aufweist, an den sich ein Mittelstück (14) anschließt, das an seinem freien Ende von einem flachen Abschlussflansch (16) abgeschlossen wird, wobei die Abmessungen des Röhrchens so ausgebildet sind, dass es auf einen entsprechend ausgebildeten spitzen Trokar (20) aufgeschoben werden kann.

## Beschreibung

Die Erfindung betrifft ein Röhrchen zur Belüftung des Mittelohrs, das auf einem Trokar befestigt werden kann, womit es möglich ist, das Röhrchen ohne vorherigen Schnitt in das Trommelfell zu setzen.

Das Ohr besteht aus dem Außenohr, dem vom Außenohr durch das Trommelfell getrennten Mittelohr und dem daran anschließenden Innenohr. Bei Erkrankungen des Mittelohrs, wie beispielsweise einer Mittelohrentzündung oder eines Tubenkatarrhs kann es sein, dass sich im Mittelohr durch Schwellungen oder Eiterbildungen ein überdruck bildet. Dieser kann zu einer schmerzhaften Dehnung des Trommelfells führen.

Als Gegenmaßnahme wird dabei in der Regel durch einen chirurgischen Eingriff ein Schnitt in das Trommelfell eingefügt. Durch diese Belüftungsöffnung kann dann ein Druckausgleich zwischen der äußeren Umgebung und dem Mittelohr stattfinden. Außerdem kann sich im Mittelohr angesammelter Eiter abfließen.

Damit die im Trommelfell durch einen Schnitt angebrachte öffnung offen bleibt, wird in diese öffnung ein Röhrchen eingesetzt.

Der Erfindung liegt die Aufgabe zugrunde, ein Röhrchen zur Belüftung des Mittelohrs anzugeben, das so ausgebildet ist, dass es zusammen mit einem entsprechend geformten Trokar, ohne dass zuvor ein Schnitt im Trommelfell angebracht wurde, in das Trommelfell gesetzt werden kann.

Die gestellte Aufgabe wird erfindungsgemäß mit dem Röhrchen zur Belüftung des Mittelohrs, das die im Hauptanspruch aufgeführten Merkmale aufweist, gelöst. Die Unteransprüche geben bevorzugte Weiterbildungen an.

Das erfindungsgemäße Röhrchen ist so ausgebildet, dass es einen vorderen kegelförmigen Flansch aufweist, an den sich ein Bereich mit einem verminderten Außendurchmesser anschließt, an den sich schließlich als Abschluss ein flacher Flansch anschließt. Das erfindungsgemäße Röhrchen wird auf einen passend ausgebildeten Trokar gesetzt. Der Trokar weist eine vordere Spitze auf, deren Verlauf bezüglich des Winkels und des Durchmessers so ausgebildet ist, dass das Röhrchen mit seinem vorderen kegelförmigen Flansch darauf geschoben werden kann. Vorzugsweise weist der Trokar in einem gewissen Abstand zu seiner vorderen Spitze einen Absatz auf. Das Röhrchen kann somit so aufgesteckt werden, dass es mit seinem hinteren flachen Anschlussflansch an diesen Absatz stößt. Es wird somit eine stets gleichbleibende wohl definierte Lage des Röhrchens auf dem Trokar erreicht.

Der Trokar wird mit dem aufgesteckten Röhrchen in das Trommelfell gestoßen. Seine Spitze durchschneidet dabei das Trommelfell. Trokar und Röhrchen werden so weit eingeschoben, bis ein Hautlappen des Trommelfells mit dem Mittelstück des Röhrchens in Verbindung kommt. Das Mittelstück des Röhrchens weist gegenüber dem maximalen Durchmesser seines vorderen kegelförmigen Flansches und auch gegenüber dem Außendurchmesser seines hinteren flachen Abschlussflansches einen verminderten Durchmesser auf. Der Hautlappen kann somit in dieses Mittelteil eingreifen und das Röhrchen halten. Anschließend wird der Trokar wieder rückwärts aus dem Trommelfell herausgezogen, wobei sich das Röhrchen von ihm löst und im Trommelfell verbleibt, da der Hautlappen des Trommelfells, der sich im Eingriff mit dem Mittelstück des Röhrchens befindet, das Röhrchen sicher hält.

Das erfindungsgemäße Röhrchen zur Belüftung des Mittelrohrs ist, um eine Korrosion oder eine Irritation der Haut zu verhindern, vorzugsweise aus Silber, Gold, Gold-Platin, Titan oder Kunststoff hergestellt, oder es weist eine Beschichtung aus Gold oder Titannitrid auf.

Der Trokar kann insgesamt geradlinig ausgebildet sein, oder er kann eine Biegung oder einen Knick aufweisen, sodass die Einsicht zum Trommelfell beim Einführen des Trokars in das Trommelfell nicht behindert wird. Vorteilhafterweise weist der Trokar an seinem hinteren Ende eine Kopplungsvorrichtung auf, mit der er an ein chirurgisches Instrument angekoppelt werden kann.

Ein Ausführungsbeispiel eines erfindungsgemäß ausgebildeten Röhrchen zur Belüftung des Mittelohrs und des dazu passend ausgebildeten Trokars wird nachfolgend anhand der beiliegenden Zeichnung erläutert. In den Zeichnungen sind gleiche Elemente in allen Zeichnungsfiguren mit den gleichen Bezugszahlen gekennzeichnet.

Es zeigen:
- Fig. 1: eine perspektivische Ansicht eines erfindungsgemäßen Röhrchens, wie es auf einem entsprechend ausgebildeten Trokar aufgesetzt ist;
- Fig. 2: eine geschnittene Seitenansicht des Röhrchens aus Fig. 1; und
- Fig. 3: eine Seitenansicht des für die Aufnahme des Röhrchens ausgebildeten Trokars aus Fig. 1.

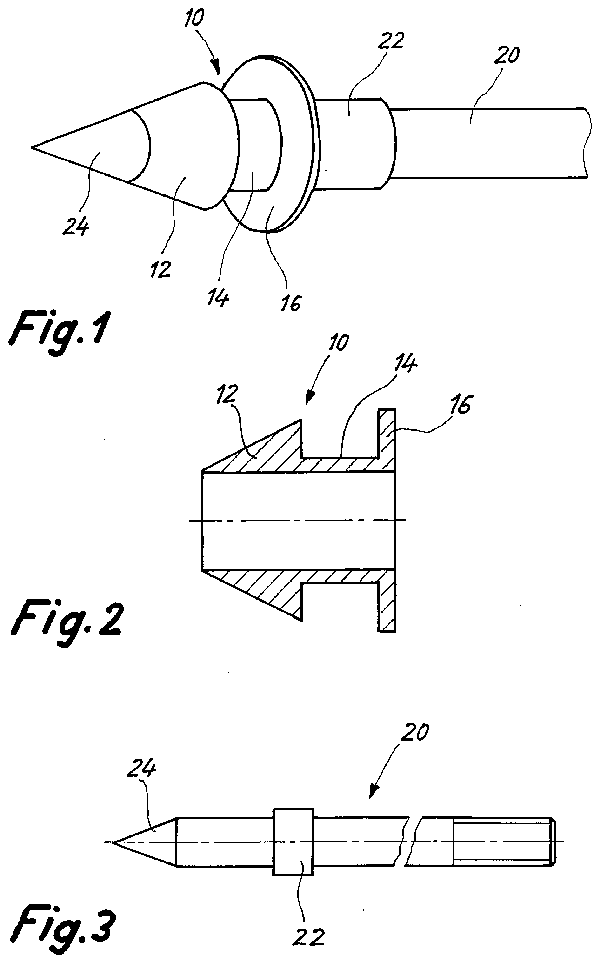

Fig. 1 zeigt eine perspektivische Ansicht eines erfindungsgemäßen Röhrchens zur Belüftung des Mittelohrs, wie es auf einen entsprechend ausgebildeten Trokar aufgesetzt ist. Das insgesamt mit der Bezugszahl 10 bezeichnete Röhrchen weist an seinem vorderen Ende einen vorderen kegelförmigen Flansch 12 auf, der eine offene Spitze besitzt. Daran schließt sich ein Mittelstück 14 des Röhrchens an. Das hintere Ende des Röhrchens 10 wird durch einen flachen Abschlussflansch 16 gebildet. Das Mittelstück 14 des Röhrchens ist so ausgebildet, dass sein Außendurchmesser kleiner ist als der maximale Außendurchmesser des vorderen kegelförmigen Flansches 12 des Röhrchens und auch kleiner als der Außendurchmesser des hinteren flachen Abschlussflansches 16.

In Fig. 1 ist auch zu erkennen, wie das erfindungsgemäße Röhrchen 10 auf einen passend ausgebildeten Trokar 20 aufgeschoben ist. Der Trokar 20 weist eine Spitze 24 auf, wobei die Maße der Spitze 24 bezüglich ihrer Abschrägung und ihres Durchmessers so ausgebildet sind, dass sie zu den Maßen des vorderen kegelförmigen Flansches 12 des Röhrchens passen. Vorteilhafterweise weist der Trokar 20, wie dies hier dargestellt ist, in einer gewissen Entfernung von seiner Spitze 24 einen Absatz 22 auf, gegen den der flache Abschlussflansch 16 des Röhrchens 10 stößt, wenn das Röhrchen in korrekter Lage auf den Trokar 20 aufgeschoben ist.

Fig. 2 zeigt eine geschnittene Seitenansicht des erfindungsgemäßen Röhrchens zur Belüftung des Mittelohrs. Man erkennt wiederum im vorderen Teil (linke Seite der Zeichnung) den vorderen kegelförmigen Flansch 12, an den sich das Mittelstück 14 mit vermindertem Außendurchmesser anschließt. An seinem hinteren Teil (rechte Seite der Zeichnung) wird das Röhrchen 10 durch den flachen Abschlussflansch 16 abgeschlossen.

Fig. 3 zeigt schließlich eine Seitenansicht des für die Aufnahme des Röhrchens ausgebildeten Trokars 20. Er weist an seinem vorderen Ende (linke Seite der Zeichnung) eine Spitze 24 auf, die bezüglich ihrer Abschrägung und ihres Außenmaßes so gestaltet ist, dass das Röhrchen 10 auf sie aufgeschoben werden kann. In einiger Entfernung von der Spitze 24 weist der Trokar vorzugsweise, wie das hier dargestellt ist, einen Absatz 22 auf, der als Anschlag für den Abschlussflansch 16 dient, wenn das Röhrchen 10 auf den Trokar 20 aufgesteckt wird.

Insgesamt beschreibt die Erfindung ein Röhrchen zur Belüftung des Mittelohrs, das so ausgebildet ist, dass es, wenn es auf einen entsprechend ausgeformten Trokar aufgeschoben wurde, mit diesem Trokar in das Trommelfell eingeschoben und dort eingesetzt werden kann, ohne dass zuvor ein Schnitt im Trommelfell ausgeführt zu werden braucht.

### Bezugszeichenliste:

- 10: Röhrchen
- 12: vorderer kegelförmiger Flansch
- 14: Mittelstück
- 16: Abschlussflansch

- 20: Trokar
- 22: Absatz des Trokars
- 24: Spitze des Trokars

## Patentansprüche

1. Röhrchen zur Belüftung des Mittelohrs, dadurch gekennzeichnet, dass es einen vorderen kegelförmigen Flansch (12) aufweist, an den sich ein Mittelstück (14) anschließt, das an seinem freien Ende von einem flachen Abschlussflansch (16) abgeschlossen wird, wobei die Abmessungen des Röhrchens so ausgebildet sind, dass es auf einen entsprechend ausgebildeten spitzen Trokar (20) aufgeschoben werden kann.

2. Röhrchen zur Belüftung des Mittelohrs nach Anspruch 1, dadurch gekennzeichnet, dass der Außendurchmesser seines Mittelstücks (14) kleiner ist als der maximale Außendurchmesser seines vorderen kegelförmigen Flansches (14) und auch kleiner als der Außendurchmesser seines hinteren Abschlussflansches (16).

3. Röhrchen zur Belüftung des Mittelohrs nach Anspruch 1 oder 2, dadurch gekennzeichnet, dass der Trokar (20) in einer Entfernung von seiner vorderen Spitze (24) einen Absatz (22) aufweist, gegen den der Abschlussflansch (16) des Röhrchens (10) stößt, wenn das Röhrchen (10) vollständig auf den Trokar (20) geschoben ist.

4. Röhrchen zur Belüftung des Mittelohrs nach einem der vorherigen Ansprüche, dadurch gekennzeichnet, dass es aus Silber, Gold, Gold-Platin, Titan oder Kunststoff besteht, oder dass es eine Beschichtung aus Gold oder Titannitrid aufweist.

5. Röhrchen zur Belüftung des Mittelohrs nach einem der vorherigen Ansprüche, dadurch gekennzeichnet, dass sein Innendurchmesser zwischen 0,5 mm und 2,0 mm liegt.

6. Röhrchen zur Belüftung des Mittelohrs nach einem der vorherigen Ansprüche, dadurch gekennzeichnet, dass es eine Länge zwischen 2 mm und 4 mm aufweist.

7. Röhrchen zur Belüftung des Mittelohrs nach einem der vorherigen Ansprüche, dadurch gekennzeichnet, dass der Trokar (20) zur besseren Sicht auf das Trommelfell gebogen ausgeführt ist oder einen Knick aufweist.

8. Röhrchen zur Belüftung des Mittelohres nach einem der vorherigen Ansprüche, dadurch gekennzeichnet, daß der Trokar (20) an seinem hinteren Ende eine Kopplungsvorrichtung aufweist, mit der er an ein chirurgisches Instrument angekoppelt werden kann.
